(19) Europäisches Patentamt
European Patent Office
Office européen des brevets

(11) Veröffentlichungsnummer : **0 009 095**
**B1**

(12) # EUROPÄISCHE PATENTSCHRIFT

(45) Veröffentlichungstag der Patentschrift : **13.01.82**

(21) Anmeldenummer : **79102587.7**

(22) Anmeldetag : **23.07.79**

(51) Int. Cl.³ : **C 07 D271/10**, C 07 D413/10, C 08 K 5/35, C 11 D 3/42, D 06 L 3/12

(54) **1,3,4-Oxadiazolon(2)-Verbindungen, Verfahren zu deren Herstellung und deren Verwendung als optische Aufheller.**

(30) Priorität : **29.07.78 DE 2833470**

(43) Veröffentlichungstag der Anmeldung : **02.04.80 (Patentblatt 80/07)**

(45) Bekanntmachung des Hinweises auf die Patenterteilung : **13.01.82 Patentblatt 82/02**

(84) Benannte Vertragsstaaten : **CH DE FR GB IT NL**

(56) Entgegenhaltungen :
**DE - A - 2 226 772**
**DE - A1 - 2 535 615**
**DE - A1 - 2 712 409**
**DE - A1 - 2 748 660**

(73) Patentinhaber : **HOECHST AKTIENGESELLSCHAFT**
**Postfach 80 03 20**
**D-6230 Frankfurt/Main 80 (DE)**

(72) Erfinder : **Prossel, Günter, Dr.**
**Fichtenweg 25**
**D-8261 Burgkirchen/Alz (DE)**
Erfinder : **Schinzel, Erich, Dr.**
**Ostpreussenstrasse 43**
**D-6238 Hofheim am Taunus (DE)**
Erfinder : **Schönberger, Norbert, Dr.**
**Am Flachsland 54**
**D-6233 Kelkheim (Taunus) (DE)**
Erfinder : **Martini, Thomas, Dr.**
**Am Schellberg 42**
**D-6232 Bad Soden am Taunus (DE)**
Erfinder : **Rösch, Günter**
**Hohlweg 17**
**D-6232 Bad Soden am Taunus (DE)**

**EP 0 009 095 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# 1,3,4-Oxadiazolon(2)-Verbindungen, Verfahren zu deren Herstellung und deren Verwendung als optische Aufheller

Aus der deutschen Offenlegungsschrift 2 748 660 sind unter anderem bereits Stilbenaufheller bekannt, die durch eine heterocyclische Gruppe in Form der Benzoxazolylgruppe und durch eine 1,2,4-Oxdiazolyl-(5)-Gruppe substituiert sind. Es wurde nun überraschend gefunden, daß derartige, durch eine heterocyclische Gruppe substituierte Stilbenaufheller in ihrer Aufhellwirkung verbessert werden können, wenn man anstelle der 1,2,4-Oxdiazolyl-(5)-Gruppe eine 1,3,4-Oxdiazolon-(2)-Gruppe einführt.

Die vorliegende Erfindung betrifft somit neue 1,3,4-Oxdiazolon-(2)-Verbindungen der allgemeinen Formel (I)

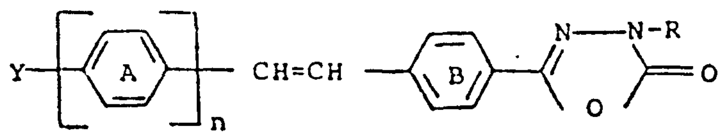

(I)

worin

Y eine Carboxy-, Cyano-, Carboalkoxy-, Carbonamido-, mono- oder di-Alkylcarbonamidogruppe oder eine Phenyl-, p-Cyanphenyl- oder p-Carbalkoxyphenylgruppe oder eine 5-Aryl-1,3,4-oxadiazolyl(2)-, 3-Aryl-1,3,4-oxadiazol-2-on-yl(5)-, 1,2,4-Triazolyl-(1)-, 1,2,3-Triazolyl(2)-, Benzofuranyl(2)-, Benzoxazolyl(2)-, Benzimidazolyl(2)-, 1,2,4-Triazolium- oder Benzimidazolium-Gruppe,

R Wasserstoff, eine Alkylgruppe oder eine Phenylgruppe, die durch 1 bis 3 Alkyl- oder Alkoxygruppen, Halogenatome oder eine Carboxy-, Cyano-, Carboalkoxy-, Carbonamido-, mono- oder di-Alkylcarbonamidogruppe substituiert sein kann, bedeutet,

n die Werte 0 und 1 annehmen kann und

die Ringe A und B noch weitere nicht chromophore Substituenten tragen können.

Gegenstand der Erfindung ist weiterhin ein Verfahren zur Herstellung der Verbindungen der Formel (1) sowie deren Verwendung als optische Aufheller.

Unter nicht-chromophoren Substituenten der Ringe A und B werden z.B. verstanden Alkyl mit 1 bis 12 Kohlenstoffatomen, Cyclohexyl, Phenylalkyl mit 1 bis 3 Kohlenstoffatomen im Alkylteil, unsubstituiertes oder mit 1 oder 2 Substituenten aus der Reihe Chlor, Methyl oder Methoxy substituiertes Phenyl, Alkoxy mit 1 bis 4 Kohlenstoffatomen, unsubstituiertes oder mit 1 oder 2 Substituenten aus der Reihe Chlor, Methyl oder Methoxy substituiertes Phenoxy, Chlor, Fluor, Brom, Cyano, —COOY, worin Y für Wasserstoff, ein salzbildendes Kation, Alkyl mit 1 bis 5 Kohlenstoffatomen oder Benzyl steht, —CONY'(Y'$_1$), worin Y' für Wasserstoff, Alkyl mit 1 bis 6 Kohlenstoffatomen, Hydroxyalkyl mit 1 bis 4 Kohlenstoffatomen, Alkoxyalkyl mit 2 bis 8 Kohlenstoffatomen, Phenyl oder Benzyl und Y'$_1$ für Wasserstoff, Alkyl mit 1 bis 6 Kohlenstoffatomen, Hydroxyalkyl mit 1 bis 4 Kohlenstoffatomen oder Alkoxyalkyl mit 2 bis 8 Kohlenstoffatomen oder Y' und Y'$_1$ zusammen mit dem Stickstoff für einen Morpholino- oder Piperidinorest stehen, oder abgewandelte Sulfogruppen wie —$SO_2$OY, worin Y die vorstehend angegebene Bedeutung hat, —$SO_2$NY'(Y'$_1$), worin Y' und Y'$_1$ die vorstehend angegebene Bedeutung haben, Alkylsulfonyl mit 1 bis 6 Kohlenstoffatomen, Benzylsulfonyl oder unsubstituiertes oder mit Chlor oder Methyl substituiertes Phenylsulfonyl, oder im Falle von zwei orthoständigen Substituenten auch Alkylen mit 3 oder 4 Kohlenstoffatomen oder 1,3-Butadienylen.

Bevorzugt sind solche Verbindungen der Formel (II)

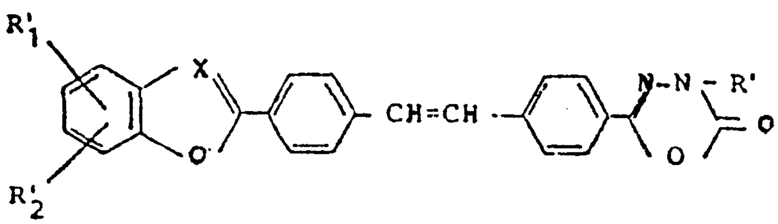

(II)

worin

X CH oder N,

R' Wasserstoff oder Alkyl, das durch eine Hydroxy-Cyano-, Carboalkoxy- oder Alkoxygruppe, ein Halogenatom, vorzugsweise Chlor, eine Dialkylamino- oder Trialkylammoniumgruppe substituiert sein kann, ferner Phenyl, das durch 1 bis 3 Alkyl- oder Alkoxygruppen oder Halogenatome, vorzugsweise Fluor oder Chlor oder eine Carboxy-, Cyano-, Carboalkoxy-, Carbonamido-, mono- oder di-Alkylcarbonamidogruppe substituiert sein kann,

bedeuten und

R'$_1$ und R'$_2$ unabhängig voneinander die gleiche Bedeutung wie R" haben und zusätzlich Alkoxy, Halogen, vorzugsweise Chlor oder Fluor, Dialkylamino oder Trialkylammonium bedeuten oder R'$_1$ und R'$_2$ gemeinsam einen Dioxymethylen-, Dioxyethylen, Alkylen oder Butadienylen bedeuten.

« Alkyl » und die davon abgeleiteten Begriffe wie Alkoxy oder Alkylen bedeuten, soweit nicht anders vermerkt, Gruppen mit 1-4 C-Atomen.

Bevorzugte Darstellungsweise der 1,3,4-Oxadiazolon(2)-Verbindungen ist die Reaktion von Säurehydraziden der allgemeinen Formel

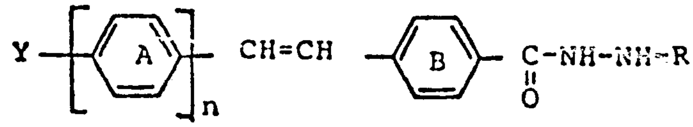

in welcher die Symbole Y, A, B, n und R die oben angegebenen Bedeutungen besitzen, in einem inerten Lösungsmittel in Gegenwart eines Säurefängers mit Phosgen oder Chlorameisensäureestern bei Temperaturen von ca. c0 bis 150 °C.

Ferner können die beanspruchten 1,3,4-Oxadiazolon(2)-Verbindungen dargestellt werden, indem man in einem inerten Lösungsmittel eine Verbindung der Formel

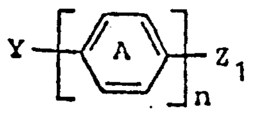

mit einer Verbindung der Formel

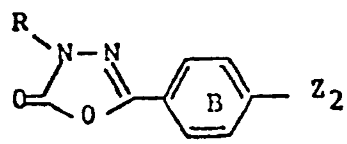

in Gegenwart eines alkalischen Kondensationsmittels umsetzt, wobei Y, R, A, B und n die oben angegebenen Bedeutungen haben und eines der Symbole $Z_1$ und $Z_2$ eine Formylgruppe und das andere eine Gruppierung der Formel

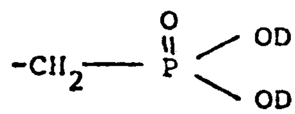

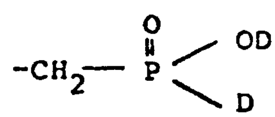

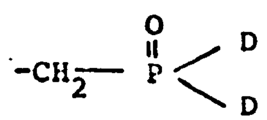

oder

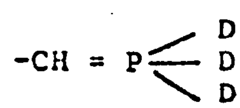

bedeuten, worin D einen unsubstituierten oder substituierten Alkylrest, einen Arylrest, einen Cycloalkylrest oder einen Aralkylrest darstellt, umsetzt.

Man führt dieses Herstellungsverfahren vorteilhaft in indifferenten Lösungsmitteln durch. Als Beispiele hierfür seien Kohlenwasserstoffe wie Toluol und Xylol oder Alkohole wie Methanol, Äthanol, Isopropanol, Butanol, Glykole, Glykoläther wie 2-Methoxyäthanol, Hexanole, Cyclohexanol und Cyclooctanol, ferner Äther wie Diisopropyläther, Tetrahydrofuran und Dioxan sowie Dimethylsulfoxyd, Formamid und N-Methylpyrrolidon genannt. Besonders geeignet sind polare organische Lösungsmittel wie Dimethylformamid und Dimethylsulfoxyd.

Die Temperatur, bei der die Umsetzung durchgeführt wird, kann in weiten Grenzen schwanken. Sie wird bestimmt durch die Beständigkeit des verwendeten Lösungsmittels gegenüber den Reaktionsteilnehmern, insbesondere gegenüber den stark basischen Alkaliverbindungen, durch die Reaktivität der Kondensationspartner und durch die Wirksamkeit der Kombination Lösungsmittel-Base als Kondensationsmittel.

In der Praxis kommen hiernach im allgemeinen Temperaturen zwischen etwa 10 und 100 °C in Betracht, insbesondere wenn Dimethylformamid oder Dimethylsulfoxid als Lösungsmittel verwendet werden. Der Temperatur-Vorzugsbereich liegt bei 20 bis 60 °C.

Als stark basische Alkaliverbindungen kommen vor allem die Hydroxyde, Amide und Alkoholate (vorzugsweise solche primärer, 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z.B. Phenyllithium oder stark basische Amine (einschließlich Ammoniumbasen) z.B. Trialkylammoniumhydroxyde, mit Erfolg verwendet werden.

Ein weiteres Verfahren zur Herstellung der erfindungsgemäßen 1,3,4-Oxadiazolon(2)-Verbindungen besteht darin, daß man in einem polaren, neutralen bis basischen Lösungsmittel und in Gegenwart einer stark basischen Alkaliverbindung eine Schiff'sche Base der allgemeinen Formel

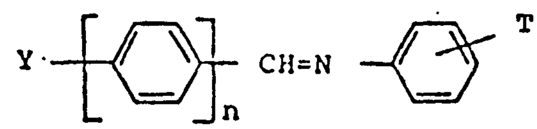

wobei Y, A und n die oben angegebene Bedeutung haben, und T Wasserstoff oder Chlor bedeutet, mit einer Methylverbindung der Formel

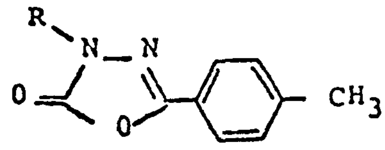

oder eine Schiff'sche Base der Formel

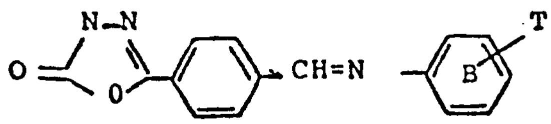

mit einer Methylverbindung der Formel

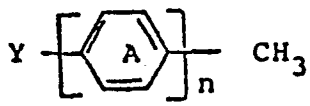

umsetzt, wobei die Symbole Y, A, B, R und n die oben angegebene Bedeutung haben und T Wasserstoff oder Chlor bedeutet.

Die methylgruppenhaltigen Verbindungen können mit den Anilen in Gegenwart eines geeignet starken polaren, neutralen bis alkalischen organischen Lösungsmittel umgesetzt werden, insbesondere solche, die frei von Wasserstoffatomen sind, die durch Alkalimetalle ersetzbar sind. In der Praxis kommen als solche Lösungsmittel vor allem Dialkylamide der Ameisensäure und der Phosphorsäure sowie Tetraalkylharnstoffe in Betracht, wobei « Alkyl » eine niedere, 1 bis 4 Kohlenstoffatome enthaltende Alkylgruppe, insbesondere eine Methylgruppe bedeutet. Als wichtige Vertreter solcher Lösungsmittel seien genannt : Diäthylformamid, Hexamethyl-phosphorsäure-triamid, Tetramethylharnstoff und insbesondere Dimethylformamid. Es kommen auch Lösungsmittelgemische in Betracht.

Für die Umsetzung ist weiterhin wie erwähnt eine stark basische Alkaliverbindung erforderlich. Je nach der Art des verwendeten Lösungsmittels und der Reaktionsfähigkeit des eingesetzten Anils sind dazu geeignet bestimmte Natriumalkoholate wie Natrium-t-butylat und besonders Kaliumverbindungen der Zusammensetzung

$$KOC_{m-1}H_{2m-1}$$

worin m eine ganze Zahl von 1 bis 6, vorzugsweise 1 bis 5, darstellt, wie z.B. Kaliumhydroxyd oder insbesondere Kalium-tertiär-butylat. Im Falle von Alkali-Alkoholaten, ist hierbei in praktisch wasserfreiem Medium zu arbeiten, während beim Kaliumhydroxyd ein geringer Wassergehalt) bis zu etwa 15 % (z.B. Kristallwassergehalt) noch erlaubt ist. Kaliumhydroxyd oder Natrium-t-butylat verwendet man zuweilen vorteilhaft in Kombination mit Hexamethylphosphorsäure-triamid bei höherer Temperatur, z.B. bei 100-130 °C an. Selbstverständlich ist es auch möglich, mit Gemischen verschiedener Basen zu arbeiten.

Die vorstehend definierten neuen Verbindungen zeigen in gelöstem oder feinverteiltem Zustand eine mehr oder weniger ausgeprägte Fluoreszenz. Sie können zum optischen Aufhellen der verschiedensten synthetischen, halbsynthetischen oder natürlichen organischen Materialien oder Substanzen, welche solche organischen Materialien enthalten, verwendet werden.

Hierfür seien beispielsweise, ohne daß durch die nachfolgende Übersicht irgendeine Beschränkung hierauf ausgedrückt werden soll, die folgenden Gruppen von organischen Materialien, soweit eine optische Aufhellung derselben in Betracht kommt, genannt :

I. Synthetische organische hochmolekulare Materialien :

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispiels-weise Polymerisate auf Basis von α,β-ungesättigten Carbonsäuren oder Derivaten solcher Carbonsäuren, insbesondere von Acrylverbindungen (wie z.B. Acrylestern, Acrylsäure, Acrylnitril, Acrylamiden un deren Derivaten oder deren Methacryl-Analoga), von Olefin-Kohlenwasserstoffen (wie z.B. Äthylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid),

b) Polymerisationsprodukte die durch Ringöffnung erhältlich sind, z.B. Polyamid vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte, deren Nachbehandlung, wie beispielsweise Polyester, insbesondere gesättigte (z.B. Äthylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiamin-adipat), Maleinatharze, Melaminharze, deren Vorkondensate und Analoga Polycarbonate, Silikone,

d) Polyadditionsprodukte wie Polyurethane (vernetzt und unvernetzt), Epoxidharze.

II. Halbsynthetische organische Materialien, z.B. Celluloseester verschiedener Veresterungsgrade (sogenanntes 2 1/2-Acetat, Triacetat) oder Celluloseäther, regenerierter Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seiden natürlichen Lackharze, Stärke, Casein.

Die optische aufzuhellendenorganischen Materialien können den verschiedenartigsten Verarbeitungszuständen (Rohstoffe, Halbfabrikate oder Fertigfabrikate) angehören. Sie können andererseits in Form der verschiedenartigsten geformten Gebilden vorliegen, d.h. beispielsweise als vorwiegend dreidimensional ausgedehnte Körper wie Platten, Profile, Spritzgußförmlinge, verschiedenartige Werkstücke, Schnitzel, Granulate oder Schaumstoffe, ferner als vorwiegend zweidimensional ausgebildete Körper wie Filme, Folien, Lacke, Überzüge Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strängen, Geweben, Gewinken, Vliesen, beflockten Substraten oder Verbundstoffen vorliegen können, erfindungsgemäß optisch aufzuhellen sind, geschieht dies mit Vorteil in wäßrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die erfindungsgemäße Veredelung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäß vorliegender Erfindung können ferner den Materialien vor order während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden :

— Zugabe zu den Ausgangssubstanzen (z.B. Monomeren) oder Zwischenprodukten (z.B. Vorkondensaten, Praepolymeren), d.h. vor order während der Polymerisation, Polykondensation oder Polyaddition,

— Aufpudern auf Polymerisatschnitzel oder Granulate für Spinnmassen,
— Badfärbung von Polymerisatschnitzel oder Granulaten für Spinnmassen,
— Dosierte Zugabe zu Spinnschmelzen oder Spinnlösungen,
— Applikation auf Spinnkabel vor dem Verstrecken.

Die neuen optischen Aufhellmittel gemäß vorliegender Erfindung können beispielsweise auch in folgenden Anwendungsformen eingesetzt werden :

a) Mischungen mit Farbstoffen (Nuancierung) oder Pigmenten (Farb- oder insbesondere z.B. Weißpigmenten) oder als Zusatz zu Färbebädern, Druck-, Ätz- oder Reservepasten. Ferner auch zur Nachbehandlung von Färbungen, Drucken oder Ätzdrucken,

b) In Mischungen mit sogenannten « Carriern », Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) In Mischung mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie « wash-and-wear », « permanent-press », « noiron »), ferner Flammfest-, Weichgriff-Schmutzablöse (« anti-soiling »)- oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditionsprodukte) in gelöster oder dispergierter Form zur Anwendung z.B. in Beschichtungs-, Imprägnier- oder Bindemitteln (Lösungen, Dispersionen, Emulsionen) für Textilien, Vliese, Papier, Leder.

e) als Zusätze zu sogenannten « master batches »,

f) als Zusätze zu den verschiedensten industriellen Produkten, um dieselben marktfähiger zu machen (z.B. Aspektverbesserungen von Seifen, Waschmitteln, Pigmenten),

g) in Kombination mit anderen, optisch aufhellend wirkenden Substanzen,

h) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Scintillatoren, für verschiedene Zwecke photographischer Art, wie z.B. für elektrophotographische Reproduktion oder Supersensibilisierung,

j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, daß der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen. So verfährt man beispielsweise bei der optischen Aufhellung einer Reihe von Fasersubstraten, z.B. von Polyesterfasern, mit den erfindungsgemäßen Aufhellern zweckmäßig in der Weise, daß man diese Fasern mit den wäßrigen Dispersionen (gegebenenfalls auch Lösungen) der Aufhellmittel bei Temperaturen unter 75 °C, z.B. bei Raumtemperatur, imprägniert und einer trockenen Wärmebehandlung bei Temperaturen über 100 °C unterwirft, wobei es sich im allgemeinen empfiehlt, das Fasermaterial vorher noch bei mäßig erhöhter Temperatur, z.B. bei mindestens 60 °C bis etwa 130 °C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einem einzigen Arbeitsgang zusammengelegt werden. Mit den erfindungsgemäßen Verbindungen werden nach dem vorstehenden Applikationsverfahren auf Polyesterfaser hervorragende Weißgrade mit sehr hoher Lichtbeständigkeit erhalten.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,005 und 2, vorzugsweise 0,1 bis 0,5 Gewichtsprozent von Interesse.

Aus verschiedenen Gründen ist es oft zweckmäßig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphate oder Alkalimetallsilikaten.

Die neuen optischen Aufhellmittel eignen sich auch als Zusätze für Waschbäder oder zu Gewerbe- und Haushaltswaschmitteln, wobei sie in verschiedener Weise zugesetzt werden können. Zu Waschbädern werden sie zweckmäßig in Form ihrer Lösungen in Wasser oder organischen Lösungsmitteln oder auch in feiner Verteilung als wäßrige Dispersionen zugegeben. Zu Haushalt- oder gewerblichen

Waschmitteln werden sie vorteilhaft in irgendeiner Phase des Herstellungsprozesses der Waschmittel, z.B. der sogenannten « Slurry » vor dem Zerstäuben dem Waschpulver oder bei der Vorbereitung flüssiger Waschmittelkombinationen zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierter Arylsulfonsäuren, Sulfocarbonsäureester mittlerer bis höherer Alkohole, Fettsäureacylaminoalkyl-, oder -aminoacrylglycerinsulfonate, Phosphorsäureester von Fettalkoholen usw. in Frage. Als Aufbaustoffe, sogenannte « Builders », kommen z.B. Alkalipoly- und -polymetaphosphate, Alkalipyrophosphate, Alkalisalze oder Carboxymethylcellulose und andere « soilredepositionsinhibitoren », ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren in Betracht. Ferner können in den Waschmitteln beispielsweise enthalten sein : antistatische Mittel, rückfettende Hautschutzmittel wie Lanolin, Enzyme, Antimikrobika, Parfüme und Farbstoffe.

## Beispiel 1

13,3 g des 4-(3-Phenyl-1,3,4-oxadiazol-2-on-yl (5))-benzyl-phosphonsäurediethylesters (80,9 %ig L. GC) und 4,9 g p-Cyanbenzaldehyd (90 %ig) werden in 40 ml Dimethylformamid gelöst und zu einer Suspension von 1,9 g Kaliumhydroxid in 40 ml DMF hinzugetropft, wobei die Temperatur unter 30° gehalten wird. Man rührt 3 Std. bei Raumtemperatur nach, saugt das ausgefallene Reaktionsprodukt ab und wäscht mit Methanol und Wasser neutral. Nach mehrmaligem Umlösen aus Chlorbenzol werden 4,0 g der Verbindung 101 vom Schmp. 247-249° in Form eines schwach gelben Kristallpulvers erhalten.

Zur Herstellung des obigen Phosphonesters werden 32,7 g des 4-(3-Phenyl-1,3,4-oxadiazol-2-on-yl (5))-benzyl-bromids in 80 ml Dimethylformamid mit 18 ml Triethylphosphit 3 Std. auf 145-150 °C erhitzt. Anschließend wird überschüssiges Triethylphosphit und das Lösungsmittel in Vakuum abdestilliert. Es verbleiben 39,4 g eines zähen Öls, dessen Gehalt an Phosphonester gaschromatographisch mit 80,9 % bestimmt wurde.

Die obige Bromverbindung wird erhalten durch Umsetzen von 25,2 g des 4-(3-Phenyl-1,3,4-oxadiazol-2-on-yl (5))-toluols mit 17,8 g N-Bromsuccinimid in 250 ml Tetrachlormethan in der Siedehitze und in Gegenwart von 0,1 g Azo-bis-isobutyronitril. Nach der üblichen Aufarbeitung werden 32,7 g der Brommethylverbindung erhalten, welche nach Umlösung aus Ethanol einen Schmp. von 112-113° zeigt.

Die obige Tolylverbindung wird gewonnen durch Umsetzung von 113,2 g Toluylsäurephenylhydrazid in 2 l Toluol mit 100 g Phosgen in Gegenwart von 200 g Triäthylamin. Dazu heitzt man die toluolische Lösung des Hydrazids, welche als Protonenfänger Triäthylamin enthält, auf 90-100° und leitet in diese Lösung die angegebene Menge Phosgen ein, wobei Triethylamin-hydrochlorid ausfällt. Man kocht 4 Std. am Rückfluß, versetzt mit Wasser und vertreibt das Toluol durch Wasserdampfdestillation. Das ungelöste Reaktionsprodukt wird abgesaugt und aus einer Mischung aus 100 ml DMF und 600 ml Methanol umkristallisiert. Man erhält 98,2 g des Oxadiazolons vom Schmp. 149-151°.

## Beispiel 2

Das 4,4'-Bis(3 Phenyl-1,3,4-oxadiazol-2-on-yl (5))-stilben (106) wird auf folgendem Wege erhalten : 8,0 4-(3 Phenyl-1,3,4-oxadiazol-2-on-yl (5)) benzaldehyd und 13,3 g 4-(3-Phenyl-1,3,4-oxadiazol-2-on-yl (5)) benzyl-phosphonester (vergl. Beispiel 1), gelöst in 40 ml Dimethylformamid, werden zu einer Suspension von 1,9 g Kaliumhydroxid in 40 ml Dimethylformamid bei einer Temperatur zwischen 20 und 30° zugetropft. Man rührt das Reaktionsgemisch 3 Std. bei Raumtemperatur nach, saugt das ausgefallene Reaktionsprodukt ab und wäscht dieses mit Methanol/Wasser neutral. Nach mehrmaligem Umlösen aus Benzoesäuremethylester unter Zusatz von Diatomeenerde werden 4,0 g der Stilbenverbindung (106) vom Schmelzpunkt 297-299° erhalten.

Zur Herstellung des obigen, substituierten Benzaldehyds werden 33,1 g des 4-(3 Phenyl-1,3,4-oxadiazol-2-onyl (5)) benzylbromids (vgl. Beispiel 1) in 200 ml Chloroform mit 14,0 g Urotropin 2 Std. am Rückfluß gekocht. Die erhaltene Substanz wird nach dem Abkühlen auf 0-5° abgesaugt, mit etwas Chloroform nachgewaschen und mit 150 ml 50 %iger Essigsäure 3 Std. am Rückfluß gekocht. Der gebildete Aldehyd wird nach dem Kaltrühren bei 5° abgesaugt, mit Wasser neutral gewaschen und getrocknet. Man erhält 14,5 g eines farblosen Kristallpulvers vom Schmp. 148-149°.

## Beispiel 3

Die Benzoxazolverbindung (111) ist auf folgenden Wege zugänglich :

Eine Lösung von 39,4 g des Phosphonesters aus Beispiel 1 und 22,3 g des Aldehyds aus Beispiel 2 in 200 ml Dimethylformamid wird zu einer Aufschlämmung von 6,2 g gemahlenen Kaliumhydroxid in 50 ml

Dimethylformamid zugetropft und durch Kühlung die Reaktionstemperatur unter 30° gehalten. Man rührt 1 Std. bei Raumtemperatur nach, saugt das ausgefallene Reaktionsprodukt ab und wäscht es zweimal mit Dimethylformamid und anschließend mit Wasser neutral. Nach mehrfacher Umlösung aus N-Methylpyrrolidon wird ein schwach gelbes Kristallpulver vom Schmp. 330-340° erhalten.

In analoger Weise können nach der Methode der PO-aktivierten Olefinierung die in der folgenden Tabelle aufgeführten erfindungsgemäßen Verbindungen hergestellt werden.

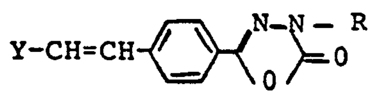

| No | R | Y | Schmp. | Adsorption λ Max (mμ) in DMF | Fluor. in DMF |
|---|---|---|---|---|---|
| 101 | 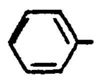 | 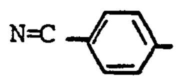 | 247-249 | 352 | blau |
| 102 | 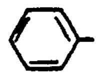 | 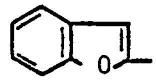 | 208-210 | 367 | blau |
| 103 | 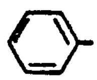 | 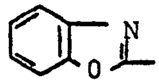 | 248-250 | 359 | blau |
| 104 | 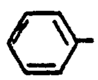 | 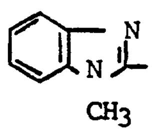 | 265-266 | 372 | grün-blau |
| 105 | 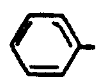 | 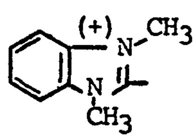 | 248-249 | 344 | blau |
| 106 | 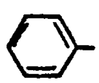 | 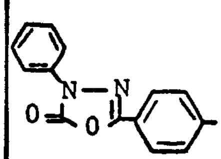 | 297-299 | 366 | blau |
| 107 | 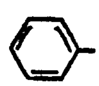 | 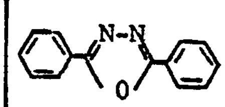 | 272-274 | 364 | violett |
| 108 | 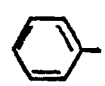 | 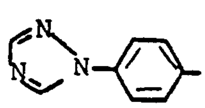 | 236-239 | 353 | blau |
| 109 | 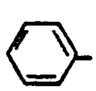 | 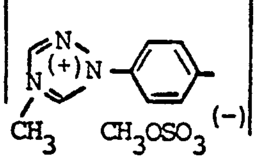 | 280-281 | 349 | blau |

| No | R | Y | Schmp. | Adsorption λ Max (mμ) in DMF | Fluor. in DMF |
|---|---|---|---|---|---|
| 110 | 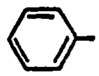 | 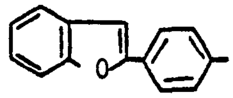 | 261-264 | 378 | grün-blau |
| 111 | 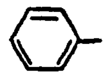 | 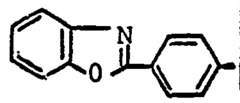 | 330-340 | 370 | violett |

Beispiel 4

Ein Gewebe aus Polyester-Stapelfaser wird mit einer 0,1 %igen Dispersion der Verbindung (111) imprägniert und anschließend zwischen Walzen auf einen Feuchtigkeitsgehalt von 85 %, bezogen auf das Warengewicht, abgequetscht. Nach der Trocknung bei 120° (20 sek) wird bei 200° 40 sek auf einem Spannrahmen thermosoliert. Das so behandelte Textilmaterial zeigt im Vergleich zur unbehandelten Ware einen hervorragenden Weißgrad.

Ähnlich gute Ergebnisse werden mit den übrigen Verbindungen der Tabelle, insbesondere den Verbindungen (106) und (107) erhalten.

**Ansprüche**

1. 2,3,4-Oxadiazolon (2)-Verbindungen der allgemeinen Formel (I)

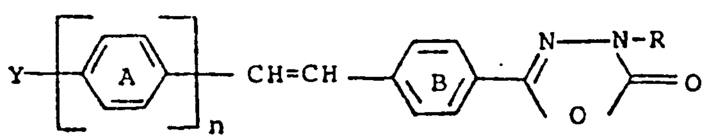

(I)

worin

Y eine Carboxy-, Cyano-, Carboalkoxy-, Carbonamido-, mono- oder di-Alkylcarbonamidogruppe oder eine Phenyl-, p-Cyanphenyl- oder p-Carbalkoxyphenylgruppe oder eine 5-Aryl-1,3,4-oxadiazolyl (2)-, 3-Aryl-1,3,4-oxadiazol-2-on-yl (5)-, 1,2,4-Triazolyl-(1), 1,2,3-Triazolyl (2)-, Benzofuranyl (2)-, Benzoxazolyl (2)-, Benzimidazolyl (2)-, 1,2,4-Triazolium- oder Benzimidazolium-Gruppe,

R Wasserstoff, eine Alkylgruppe oder eine Phenylgruppe, die durch 1 bis 3 Alkyl- oder Alkoxygruppen, Halogenatome oder eine Carboxy-, Cyano-, Carboalkoxy-, Carbonamido-, mono- oder di-Alkylcarbonamidogruppe substituiert sein kann, bedeutet,

n die Werte 0 und 1 annehmen kann und

die Ringe A und B noch weitere nicht chromophore Substituenten tragen können.

2. 1,3,4-Oxadiazolon (2)-Verbindungen nach Anspruch 1 der allgemeinen Formel (II)

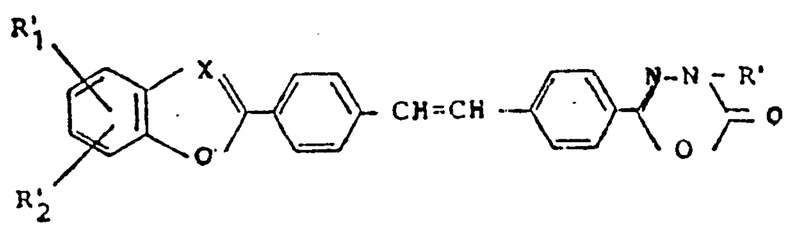

(II)

worin

X CH oder N,

R' Wasserstoff oder Alkyl, das durch eine Hydroxy-Cyano-, Carboalkoxy- oder Alkoxygruppe oder ein Halogenatom, vorzugsweise Chlor, eine Dialkylamino- oder Trialkylammoniumgruppe substituiert sein kann, ferner Phenyl, das durch 1 bis 3 Alkyl- oder Alkoxygruppen oder Halogenatome, vorzugsweise Fluor und Chlor, oder durch eine Carboxy-, Cyano-, Carboalkoxy-, Carbonamido-, mono- oder di-Alkylcarbonamidogruppe substituiert sein kann,

bedeuten und

$R'_1$ und $R'_2$ unabhängig voneinander die gleiche Bedeutung wie R' haben und zusätzlich Alkoxy, Halogen, vorzugsweise Chlor oder Fluor, Dialkylamino oder Trialkylammonium bedeuten oder $R'_1$ und $R'_2$ gemeinsam einen Dioxymethylen-, Dioxyethylen, Alkylen oder Butadienylen bedeuten.

3. Verfahren zur Herstellung von 1,3,4-Oxadiazolon (2)-Verbindungen der allgemeinen Formel 1 gemäss Anspruch 1 dadurch gekennzeichnet, daß man Säurehydrazide der allgemeinen Formel

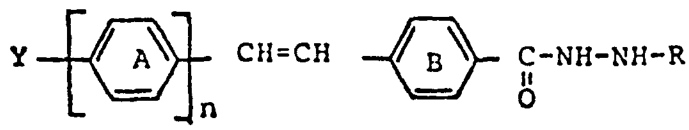

in welcher die Symbole Y, A, B, n und R die oben angegebenen Bedeutungen besitzen, in einem inerten Lösungsmittel in Gegenwart eines Säurefängers mit Phosgen oder Chlorameisensäureestern umsetzt.

4. Verfahren zur Herstellung von 1,3,4-Oxadiazolon (2)-Verbindungen nach Anspruch 1-2, dadurch gekennzeichnet, daß man in einem inerten Lösungsmittel eine Verbindung der Formel

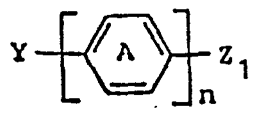

mit einer Verbindung der Formel

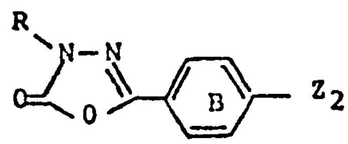

in Gegenwart eines alkalischen Kondensationsmittels umsetzt, wobei Y, R, A, B und n die im Anspruch 1 angegebenen Bedeutungen haben und eines der Symbole $Z_1$ und $Z_2$ eine Formylgruppe und das andere eine Gruppierung der Formel

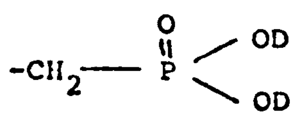

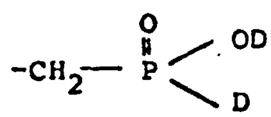

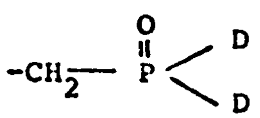

oder

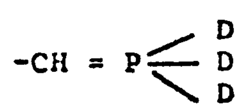

bedeuten, worin D einen unsubstituierten oder substituierten Alkylrest, einen Arylrest, einen Cycloalkylrest oder einen Aralkylrest darstellt.

5. Verfahren zur Herstellung von 1,3,4-Oxadiazolon (2)-Verbindungen nach Anspruch 1-2, dadurch gekennzeichnet, daß man in einem polaren, neutralem bis basischem Lösungsmittel und in Gegenwart einer stark basischen Alkaliverbindung eine Schiff'sche Base der allgemeinen Formel

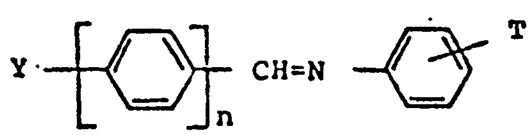

wobei Y, A und n die oben angegebene Bedeutung haben und T Wasserstoff oder Chlor bedeutet, mit einer Methylverbindung der Formel

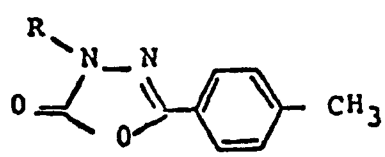

oder eine Schiff'sche Base der Formel

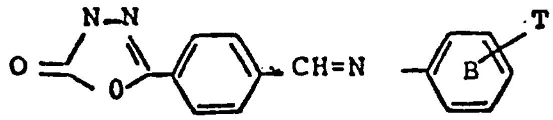

mit einer Methylverbindung der Formel

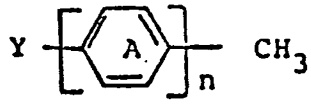

umsetzt, wobei die Symbole Y, A, B, R und n die oben angegebene Bedeutung haben und T Wasserstoff oder Chlor bedeutet.

6. Verwendung der 1,3,4-Oxadiazolon-(2)-Verbindungen nach Anspruch 1-2 als optische Aufheller.

**Claims**

1. 1,3,4-Oxadiazolone-(2) compounds of the general formula (I)

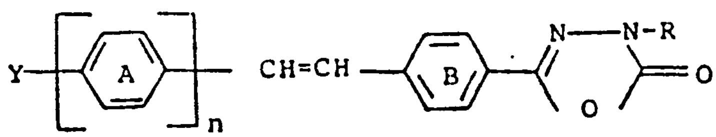

(I)

wherein

Y denotes carboxy, cyano, carboalkoxy, carbonamido, mono- or di-alkylcarbonamido or phenyl, p-cyanophenyl or p-carboalkoxyphenyl or a 5-aryl-1,3,4-oxadiazolyl (2)-, 3-aryl-1,3,4-oxadiazol-2-on-yl (5)-, 1,2,4-triazolyl-(1)-, 1,2,3-triazolyl (2)-, benzofuranyl (2)-, benzoxazolyl (2)-, benzimidazolyl (2)-, 1,2,4-triazolium- or benzimidazolium group,

R denotes hydrogen, alkyl or phenyl which may be substituted by 1 to 3 alkyl or alkoxy groups or halogen atoms or by a carboxy, cyano, carboalkoxy, carbonamido, mono- or di-alkylcarbonamido group,

n denotes 0 or 1 and

the rings A and B carry optionally further non-chromophoric substituents.

2. 1,3,4-Oxadiazolone-(2) compounds as claimed in claim 1 of the formula (II)

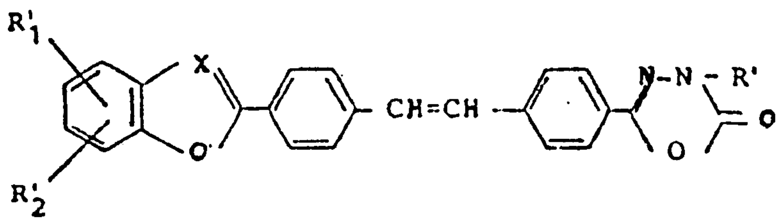

(II)

wherein

X denotes CH or N,

R' denotes hydrogen or alkyl, optionally substituted by hydroxy, cyano, carboalkyl or alkoxy, halogen, preferably chlorine, dialkylamino or trialkylammonium ; phenyl optionally substituted by 1 to 3 alkyl or alkoxy groups or by halogen atoms, preferably fluorine or chlorine or by a carboxy, cyano, carboalkoxy, carbonamido, mono- or dialkylcarbonamido group, and

$R'_1$ and $R'_2$ independent from one another have the same meaning as R' and additionally may denote alkoxy, halogen, preferably chlorine or fluorine, dialkylamino or trialkyl ammonium or $R'_1$ and $R'_2$ denote, when taken together, dioxymethylene, dioxyethylene, alkylene or butadienylene.

3. A process for the manufacture of 1,3,4-oxadiazolone-(2) compounds of the formula 1 of claim 1 which comprises reacting acid hydrazides of the formula

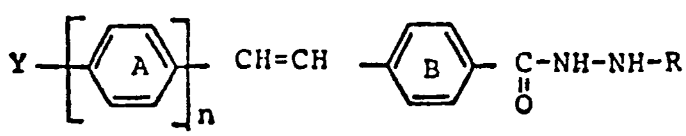

wherein Y, A, B, n and R are as defined above, in an inert solvent in the presence of an acid scavenger with phosgene or chloroformates.

4. The process for the manufacture of 1,3,4-oxadiazolone-(2) compounds as claimed in claim 1 to 2, which comprises reacting in an inert solvent a compound of the formula

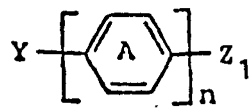

with a compound of the formula

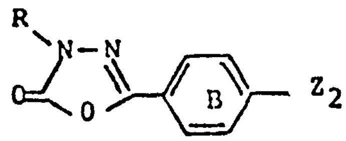

in the presence of an alkaline condensation agent, in the above formulae Y, R, A, B and n being as defined in claim 1 and one of the symbols $Z_1$ and $Z_2$ denoting formyl and the other one denoting a grouping of the formula

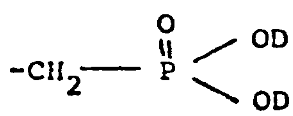

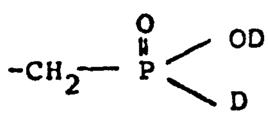

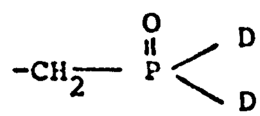

or

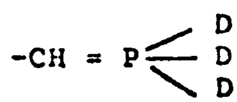

wherein D is unsubstituted or substituted alkyl ; aryl ; cycloalkyl or aralkyl.

5. The process for the manufacture of the 1,3,4-oxadiazolone-(2) compounds as claimed in claims 1 to 2, which comprises reacting in a polar, neutral to basic solvent and in the presence of a highly basic alkali metal compound, a Schiff's base of the formula

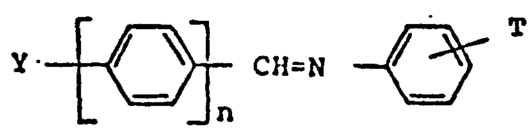

wherein Y, A and n are as defined above and T denotes hydrogen or chlorine, with a methyl compound of the formula

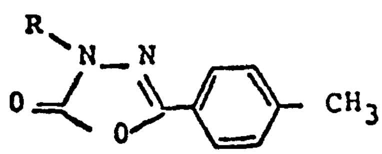

or reacting a Schiff's base of the formula

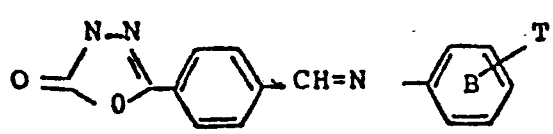

with a methyl compound of the formula

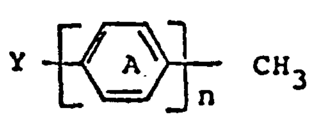

wherein the symbols Y, A, B, R and n are as defined above and T denotes hydrogen or chlorine.

6. Use of the 1,3,4-oxadiazolon-(2) compounds of claims 1 to 2 as optical brighteners.

**Revendications**

1. Composés contenant un groupe oxo-2 oxadiazole-1,3,4 yle, caractérisés en ce qu'ils répondent à la formule générale :

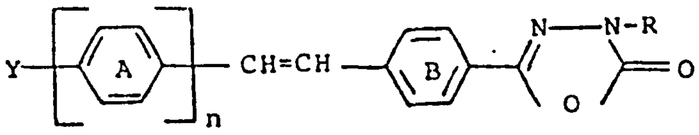

(I)

dans laquelle :

Y est un groupe carboxyle, cyano, alcoxycarbonyle, carbamoyle, mono- ou di-alkylcarbamoyle ou un groupe phényle, p-cyanophényle ou p-alcoxycarbonylphényle ou un groupe aryl-5 oxadiazole-1,3,4 yle-2, oxo-2 aryl-3 oxadiazole-1,3,4 yle-5, triazole-1,2,4 yle-1, triazole-1,2,3 yle-2, benzofurannyle-2, benzoxazolyle-2, benzimidazolyle-2, triazolium-1,2,4 ou benzimidazolium ;

R est un atome d'hydrogène, un groupe alkyle ou un groupe phényle qui peut être substitué par un à trois groupes alkyles ou alcoxy, atomes d'halogène ou par un groupe carboxyle, cyano, alcoxycarbonyle, carbamoyle, mono- ou di-alkylcarbamoyle ;

n est un nombre valant 0 ou 1 ; et

les cycles A et B peuvent porter encore d'autres substituants non chromophores.

2. Composés comportant un groupe oxo-2 oxadiazole-1,3,4 yle selon la revendication 1, caractérisés en ce qu'ils répondent à la formule générale (II) :

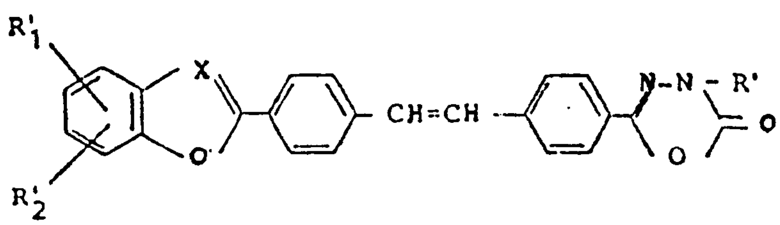

(II)

dans laquelle :

X représente CH ou N,

R' est un atome d'hydrogène ou un groupe alkyle pouvant être substitué par un groupe hydroxyle, cyano, alcoxycarbonyle ou alcoxy ou un atome d'halogène, de préférence le chlore, ou par un groupe dialkylamino ou trialkylammonium ; R' peut également représenter un groupe phényle, pouvant être substitué par un à trois groupes alkyle ou alcoxy ou atomes d'halogène, de préférence le fluor et le chlore, ou par un groupe carboxyle, cyano, alcoxycarbonyle, carbamoyle, mono- ou di-alkyl-carbamoyle ; et

$R'_1$ et $R'_2$, indépendamment l'un de l'autre, ont le même sens que R' et peuvent en outre représenter un groupe alcoxy, un atome d'halogène, de préférence le chlore ou le fluor, un groupe dialkylamino ou trialkylammonium, ou bien $R'_1$ et $R'_2$ pris ensemble peuvent représenter un groupe dioxyméthylène, dioxyéthylène, alkylène ou butadiénylène.

3. Procédé pour fabriquer des composés comportant un groupe oxo-2 oxadiazole-1,3,4 yle de formule générale (I) selon la revendication 1, procédé caractérisé en ce qu'on fait réagir des hydrazides d'acides de formule générale :

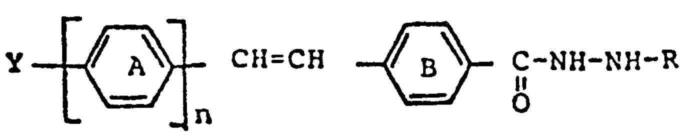

dans laquelle les symboles Y, A, B, n et R ont les sens précités dans un solvant inerte en présence d'un accepteur d'acide avec du phosgène ou avec des esters de l'acide chloroformique.

4. Procédé pour produire des composés comportant un groupe oxo-2 oxadiazole-1,3,4 yle selon la revendication 1 ou 2, caractérisé en ce qu'on fait réagir dans un solvant inerte un composé de formule :

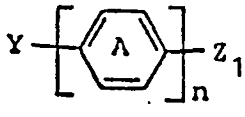

avec un composé de formule :

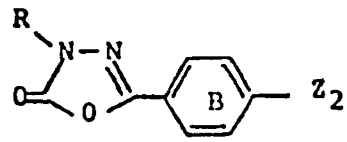

en présence d'un agent alcalin de condensation, formules dans lesquelles Y, R, A, B et n ont les sens indiqués à la revendication 1, et l'un des symboles $Z_1$ et $Z_2$ est un groupe formyle et l'autre représente un groupement de formule :

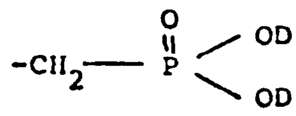

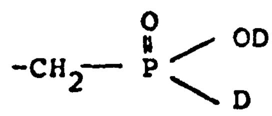

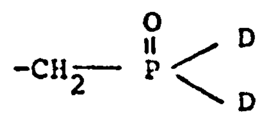

ou

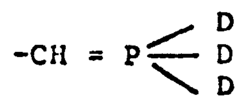

où D représente un radical alkyle non substitué ou substitué, un radical aryle, un radical cycloalkyle ou un radical aralkyle.

5. Procédé pour produire des composés comportant un groupe oxo-2 oxadiazole-1,3,4 yle selon la revendication 1 ou 2, caractérisé en ce qu'on fait réagir dans un solvant polaire, neutre à basique et en présence d'un composé alcalin fortement basique, une base de Schiff de formule générale :

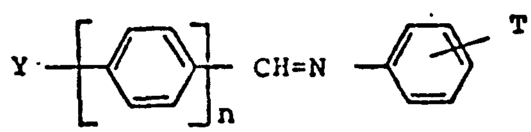

dans laquelle Y, A et n ont le sens précité ; et T est un atome d'hydrogène ou de chlore avec un composé méthylé de formule :

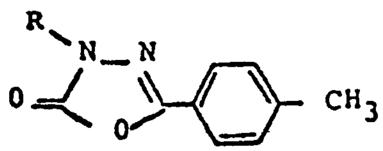

ou bien l'on fait réagir une base de Schiff de formule :

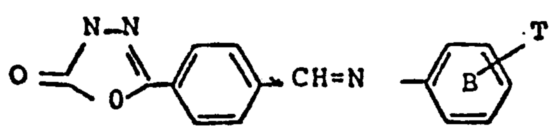

avec un composé méthylé de formule :

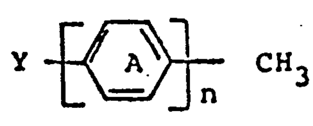

formules dans lesquelles les symboles Y, A, B, R et n ont le sens précité, et T est un atome d'hydrogène ou de chlore.

6. Application des composés comportant un groupe oxo-2 oxadiazole-1,3,4 yle selon l'une des revendications 1 et 2 comme agent d'éclaircissement optique.